# EUROPEAN PATENT APPLICATION

(11) **EP 4 817 674 A1**
(43) Date of publication of application: **07.10.2026**
(21) Application number: 26170512.3
(22) Date of filing: 02.04.2026
(51) Int. Cl.: A61N 1/378, H01F 27/245, H02J 50/00, H02J 50/10

(54) **MEDICAL WIRELESS CHARGING DEVICE WITH FERRITE STACK CENTERED AND DIRECTLY CONTACTED TO A CHARGING COIL**

(30) Priority: 04.04.2025 US 202563783290 P; 01.04.2026 US 202619636325
(71) Applicant: Greatbatch Ltd., Clarence, NY 14031 (US)
(72) Inventor: CURIEL, Rocio Belen, 11300 Montevideo (UY); BARBAT, Gabriel Amalcar, 11300 Ciudad de la Costa (UY); SIRIO, Lucia L., 11700 Montevideo (UY)
(74) Representative: Mathys & Squire

(57) **Abstract**

A wireless charger for an implantable medical device is described. The wireless charger has a housing forming an internal compartment. A printed circuit board (PCB) assembly disposed inside the housing comprises at least one electronic component that is configured to control the various functions of the wireless charger, and a battery is connected to the PCB assembly to power the at least one electronic component. A wound charging coil inside the housing is configured to output inductive power to charge the battery powering a spaced-apart implanted medical device. There is also a stack of at least two ferrite sheets that is directly contacted to the charging coil.

## Description

### CROSS-REFERENCE TO RELATED APPLICATION

This application claims priority to U.S. provisional application Serial No. 63/783,290, filed on April 4, 2025, as well as US Patent Application Serial No. 19/636,325, filed on 1 April 2026.

### BACKGROUND OF THE INVENTION

### 1. Field of the Invention

The present invention generally relates to a charger for an implantable medical device. More specifically, the present invention relates to a charger for wirelessly charging a medical device that has been implanted in a patient. The wireless charger addresses the problem of inefficient wireless power transfer to the implanted medical device through inductive coupling.

### 2. Prior Art

U.S. Pub. No. 2021/0194289 describes a medical system comprising a charger for an implanted medical device. The charger includes a recharge coil assembly comprising an insulative bobbin having opposed first and second major surfaces, a recharge coil, a telemetry coil, and a flux guide. The recharge coil is disposed on the first major surface of the insulative bobbin and coupled to recharger electronic components to form a resonant recharge tank circuit to power a secondary coil in the spaced-apart implanted medical device via inductive power transfer. A telemetry coil is disposed concentric to the recharge coil on the first major surface of the insulative bobbin and is also operably coupled to the recharger electronic components to form a resonant telemetry tank circuit to provide inductive telemetry with the implanted medical device. A flux guide comprising a ferrite sheet is supported on the second major surface of the insulative bobbin and positioned between the recharge and telemetry coils and a main printed circuit board assembly. This publication specifically states that the "flux guide 240 is of a size to cover both the recharge coil and the first telemetry coil 230 and can be circular, or generally circular in shape."

While the medical system described in the '289 publication can improve charging efficiency to some extent, providing the flux guide 240 with a size that is large enough to cover both the recharge coil and the first telemetry coil 230 is believed to detract from maximizing power transfer to the implanted medical device.

Therefore, there is a need to overcome the drawbacks inherent in the medical system described in the '289 publication by providing a wireless charging device utilizing a stack of multiple ferrite layers or sheets that are positioned directly above the charging coil and optionally beneath the battery. Importantly, the wireless charger of the present invention fills this need. That is done for example by providing the diameter of the stack of ferrite sheets being less than the diameter of the charging coil, which leaves an outer portion of the charging coil uncovered by the ferrite sheets. This helps to increase the quality factor of the charging coil because the coil is not in contact with most of the winding area, thereby reducing power losses that can result when the charging coil is close to the ferrite stack. The result is increased efficiency of wireless power transfer to the implanted medical device.

### SUMMARY OF THE INVENTION

Aspects and embodiments of the present invention are set out in the appended claims.

These and other aspects and embodiments useful in understanding the claimed invention are also described herein.

Any feature in one aspect of the invention may be applied to other aspects of the invention, in any appropriate combination.

These and other aspects of the present invention will become increasingly more apparent to those skilled in the art by reference to the following detailed description and to the appended drawings.

### BRIEF DESCRIPTION OF THE DRAWINGS

FIG. 1 is simplified depiction of a wireless charger 10 according to the present disclosure that is positioned on a patient P to charge a medical device 100 that is implanted in the patient's chest.
FIG. 2 is a perspective view of the wireless charger 10 depicted in FIG. 1.
FIG. 3 is an exploded perspective view showing the internal components of the wireless charger illustrated in FIG. 2.
FIG. 4 is a cross-sectional view taken along line 4-4 of the wireless charger 10 shown in FIG. 2.
FIG. 5 is a cross-sectional view that is similar to the view shown in FIG. 4 but with the stack of ferrite sheets 60 disposed inside the central opening of a donut-shaped charging coil 59.

### DETAILED DESCRIPTION OF THE PREFERRED EMBODIMENTS

A wireless charger of the present disclosure includes a plurality of thin ferrite layers or sheets, for example, from at least two to twenty, or more, ferrite sheets that are stacked directly on top of the charging coil. The stacked ferrite sheets serve to focus and optimize the induction produced magnetic field and the coil's quality factor, thereby reducing energy loss and improving the overall efficiency of wireless power transfer to a spaced-apart implanted medical device. This wireless charging improvement is incorporated into a battery powered, wireless, wearable charger that provides a convenient and portable solution for charging a spaced-apart implanted medical device, for example, an implanted pulse generator. Preferably, the charger also includes an adhesive patch, an adhesive surface, an adhesive substance, and the like. The adhesive member allows the wireless charger to be worn by a patient for hand-free charging of their implanted medical device while on the move without compromising or significantly impacting patient comfort.

In an exemplary embodiment, the wireless charger includes a stack of at least two or more ferrite sheets, preferably circular-shaped ferrite sheets, that are stacked directly above and in contact with a charging coil that is housed inside the charger to facilitate a smaller device. Preferably, the wireless charger of the present disclosure can be controlled and monitored through an app on a mobile device and its reduced size means that the charger can be placed almost anywhere on the body of a patient using a belt or an adhesive member to provide a secure and comfortable charging experience.

Furthermore, the charger can be wirelessly connected to a mobile device such as a smart phone or other cellular telephone, a smart tablet, a computer, and the like, via an app on the mobile device. This provides both the patient and the care giver with the ability to monitor alignment of the wireless charger with the implanted medical device, to monitor the charge status of the implanted medical device, and to control and monitor therapy settings and other parameters of the implanted medical device.

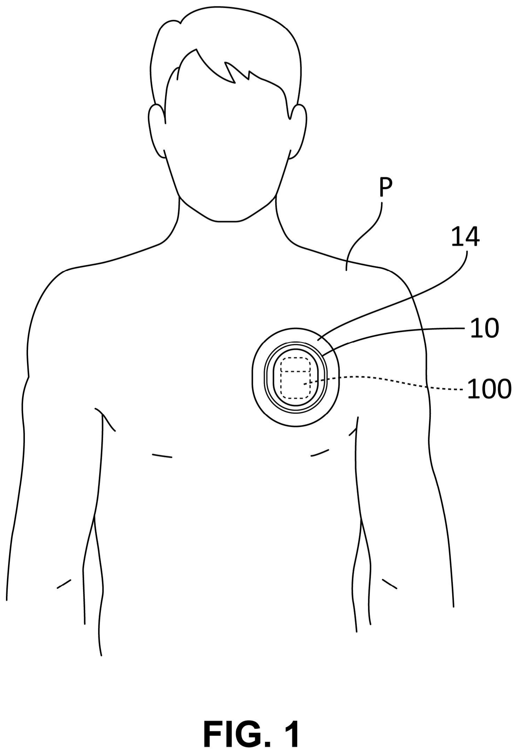

Referring now to the drawings, FIG. 1 illustrates a wireless charger 10 that is configured to charge a medical device 100 that is implanted in a patient P. The wireless charger 10 is attached to the patient P, for example, to the skin of the patient in alignment with the implanted medical device 100 to charge the medical device. The implanted medical device 100 can be, for example, a cardiac implantable pulse generator, a pacemaker, an implantable defibrillator, a neurostimulator, a cardiac monitor, a cochlear implant, and the like. Although shown implanted within the chest of the patient P, the medical device 100 can be implanted at various locations in the patient, including, but not limited to, a chest, a skull, a limb, a back, a pelvic region, and the like. The wireless charger 10 is configured to be selectively attached to the patient aligned with the medical device 100 to charge the medical device. The implantable medical device 100 is preferably implanted no more than about 4 cm deep into the patient's body.

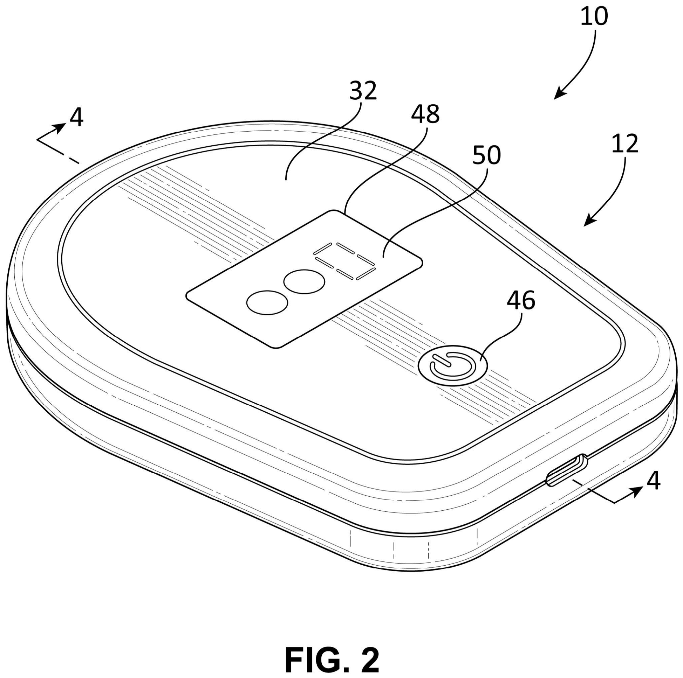

Referring now to FIGS. 2 to 4, the charger 10 is a wireless charger, which means that no wires, cords, or cables are needed to attach the charger to an external power source while the charger 10 is attached to the patient P and positioned to wirelessly charge the implanted medical device 100. In that respect, the wireless charger 10 is a relatively small and compact device that allows for increased mobility while the patient P is wearing the charger 10 to charge the medical device 100.

The wireless charger 10 includes a housing 12 providing an internal compartment and an adhesive member 14 (FIG. 1) comprising an adhesive patch or other substrate that is configured to adhere the charger to the patient during charging of the implanted medical device 100. For example, the adhesive member 14 is configured to adhere to both the charger 10 and the patient. In an exemplary embodiment, the adhesive member 14 is substantially ring-shaped to fit around the housing 12 so that the housing resides in an opening in the adhesive member adhered to the patient. In an exemplary embodiment, the housing 12 measures about 116 mm x about 87 mm x about 16 mm.

The housing 12 is comprised of a bottom clamshell-shaped member 16 that is connected to an upper clamshell-shaped member 18. The bottom clamshell 16 is comprised of a bottom wall 20 having a bottom surface that is configured to contact the skin of the patient. The bottom wall 20 meets an upwardly extending annular sidewall 22 that has an annular rim 24 spaced from the bottom wall 20. Similarly, the upper clamshell 18 is comprised of an ergonomically-shaped upper wall 26 that meets a downwardly depending annular sidewall 28 that has an annular rim 30 spaced from the upper wall 26. The upper wall 26 has a depression into which a face plate 32 fits.

The upwardly extending sidewall 22 of the bottom clamshell 16 and the downwardly depending sidewall 28 of the upper clamshell 18 include opposed recesses 34A and 34B that extend into their respective rims 24 and 30. These recess 34A, 34B form a notch that receives a charging flange 36 that surrounds and defines a charging port 38 extending outwardly from a printed circuit board (PCB) assembly 40.

The PCB assembly 40 comprises a PCB that supports at least one, but preferably a plurality of electronic components 42 which control the various functions of the wireless charger 10. The PCB components preferably include a step-up converter, a class D amplifier, microcontrollers, a Bluetooth module, and power regulation components to ensure an efficient and controlled charging process.

The charging port 38 is configured for selectively plugging in a charging cable (not shown) when a rechargeable battery 44 as a power source for charger 10 is to be charged. The charging port 38 can have various configurations, including, but not limited to, a USB port, a USB-C port, a mini-USB port, a micro-USB port, a custom port, and the like. The PCB assembly 40 also has a cut-out that is sized and shaped to provide space for the rechargeable battery 44. Preferably, the battery 44 is a lithium-ion electrochemical cell comprising a carbon-based or Li₄Ti₅O₁₂-based anode and a lithium metal oxide-based cathode, such as of LiCoO₂ or lithium nickel manganese cobalt oxide (LiNiₐMn_{b}Co_{1-a-b}O₂).

The face plate 32 that fits into the depression in the upper wall 26 of the upper clamshell 18 is preferably snapped into engagement with the upper wall and includes a power button 46 connected to the PCB assembly 40. The power button 46 allows the patient P to power the charger 10 on and off. The power button 46 can be capacitive and include a light or other indicator to denote when the wireless charger 10 is powered on. Further, the faceplate 32 includes a window 48 or opening through which the patient can view a display 50 disposed in the window. The display 50 is connected to the PCB assembly 40 by a flex-cable 52 and is configured to show various information to the patient P or caregiver, for example, charge level, charge time, an error indicator, a warning indicator, a temperature indicator, an alignment indicator, and the like.

The rechargeable battery 44 is nested in the upper clamshell 18 between spaced-apart depending interior walls 54 and 56. Interior wall 54 is spaced closer to the depending sidewall 28 of the upper clamshell 18 than interior wall 56. Interior wall 54 includes a distal inwardly-facing ledge 54A upon which an edge of the rechargeable battery 44 rests when the battery is snap-fit into position between the interior walls 54, 56.

A charging coil 58 disposed in the internal compartment of the housing is configured to output inductive power to charge the battery (not shown) that powers the spaced-apart implanted medical device 100. The charging coil 58 is configured to inductively transfer power from about 6.78 MHz to about 13.56 MHz, preferably at about 6.78 MHz, to the battery of the implanted medical device 100. The charging coil 58 includes an adhesive on the side that is adhered to the inner surface of the bottom wall 20 of the bottom clamshell 16.

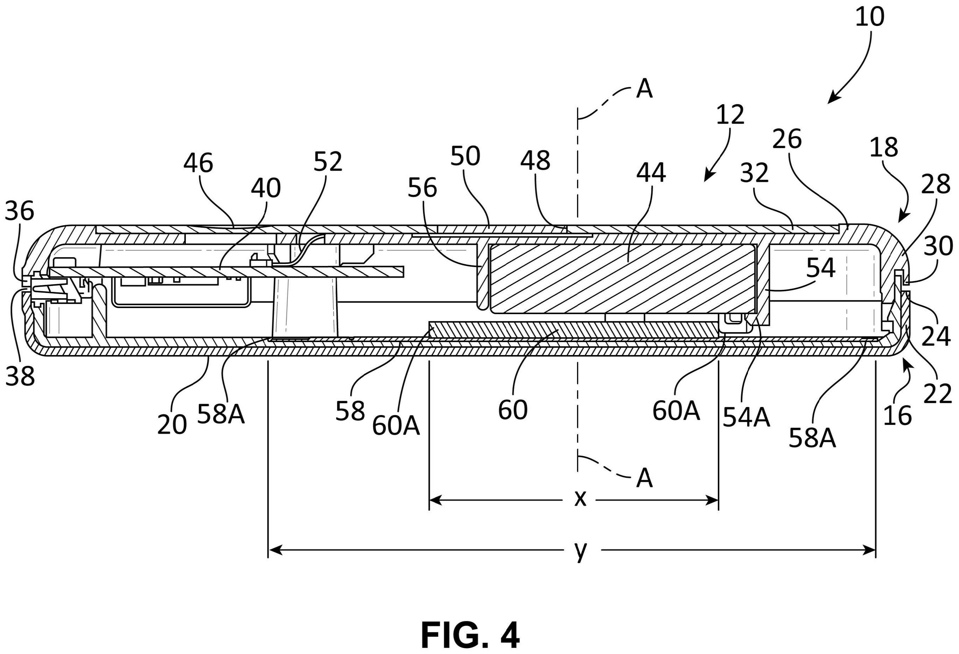

In the exemplary embodiment shown in FIG. 4, the charging coil 58 has at least two to six winds or turns, preferably three to five turns, more preferably four turns, of a copper trace, for example, a 2 oz/ft² copper trace, formed in a flexible printed circuit board 58A so that the coil has a circular shape in plan view with an outer perimeter edge 58A that defines a diameter of the coil. A longitudinal axis A-A is aligned in a co-axial relationship with the charging coil 58. A portion of the charging coil 58 is spaced below the PCB assembly 40. This means that the electrical connection from the charging coil 58 to the PCB assembly 40 is relatively short, which helps prevent unwanted eddy currents that could adversely affect recharging the implanted medical device 100. The charging coil 58 is also spaced below battery 44. The flexible printed circuit board 58A supporting the charging coil 58 is separate from the printed circuit board of the PCB assembly 40.

As further shown in FIG. 4, a stack of ferrite sheets 60 is aligned along a longitudinal axis A-A and centered on top of and in direct contact with the charging coil 58. The number of ferrite sheets 60 in the stack preferably ranges from at least two to twenty, or more. In an exemplary embodiment, there are eight ferrite sheets 60 is the stack. Each ferrite sheet 60 in the stack is formed from a porous NiCuZn ferrite sheet that is about 0.3 mm thick and has its own adhesive. The sheets are adhered together so that the stack has a thickness that ranges from about 2.0 mm to about 3.0 mm, preferably about 2.5 mm, extending along the longitudinal axis A-A. In one embodiment, the ferrite sheets 60 have a circular shape in plan view with an outer perimeter edge 60A that defines a diameter of the stack. In an exemplary embodiment, the outer diameter of the stack of ferrite sheets 60 is about 37 mm (radius = 18.5 mm). However, it is within the scope of the present disclosure that a ferrite sheet 60 comprising the stack can be in the shape of a circular disc, have a square shape, a rectangular shape, have a multi-sided shape, and the like.

It is noted that a single ferrite sheet that has the requisite thickness ranging from about 2.0 mm to about 3.0 mm is suitable for the present wireless charger 10. However, such ferrite sheets are not generally commercially available in materials that are suitable for the present charger 10. That is because their losses are too high at 6.78 MHz or 13.56 MHz and is the reason that at least two ferrite sheets are used to form the stack of ferrite sheets 60. In addition to improving magnetic coupling to the implanted device 100, having more than one ferrite layer increases the quality factor of the charging coil 58.

A preferred aspect of the present charger 10 is that the diameter of the stack of ferrite sheets 60 is less than the diameter of the charging coil 58. In an exemplary embodiment, the charging coil 60 has a diameter of about 72 mm (radius = 36 mm). With the stack of ferrite sheets 60 having a diameter of about 37 mm, the area of ferrite stack is about 1,075 mm² and the area of the charging coil 60 is about 4,072 mm² giving a coil to ferrite stack area ratio of about 3.8. This means that there is an outer portion of the charging coil 58 extending inwardly from the outer edge 58A of the coil to the outer edge 60A of the ferrite sheet stack 60 that is uncovered by the ferrite sheets. This helps to increase the quality factor of the charging coil 58 because the coil is not in contact with most of the winding area, thereby reducing losses caused by the proximity, which increases efficiency of wireless power transfer.

FIG. 4 further illustrates this aspect of the present charger in an exemplary embodiment with the charging coil 58 having a generally circular shape with an outer edge having a diameter indicated by designation "x" and with the stack of ferrite sheets 60 having a circular shape with an outer edge 60A having a diameter indicated by designation "y". That way, the charging coil to ferrite stack area ratio ranges from about 2 to 5, more preferably from about 3 to 4.

Another preferred aspect of the present wireless charger is that the stack of ferrite sheets 60 is aligned along the longitudinal axis A-A between the rechargeable battery 44 and the charging coil 58. That way, the stack of ferrite sheets 60 helps to optimize the magnetic flux emanating from the charging coil 58 to concentrate the flux more effectively in the direction of the implanted medical device 100. The ferrite stack 60 minimizes parasitic losses, ensuring that power transfer to the implanted medical device 100 is maximized.

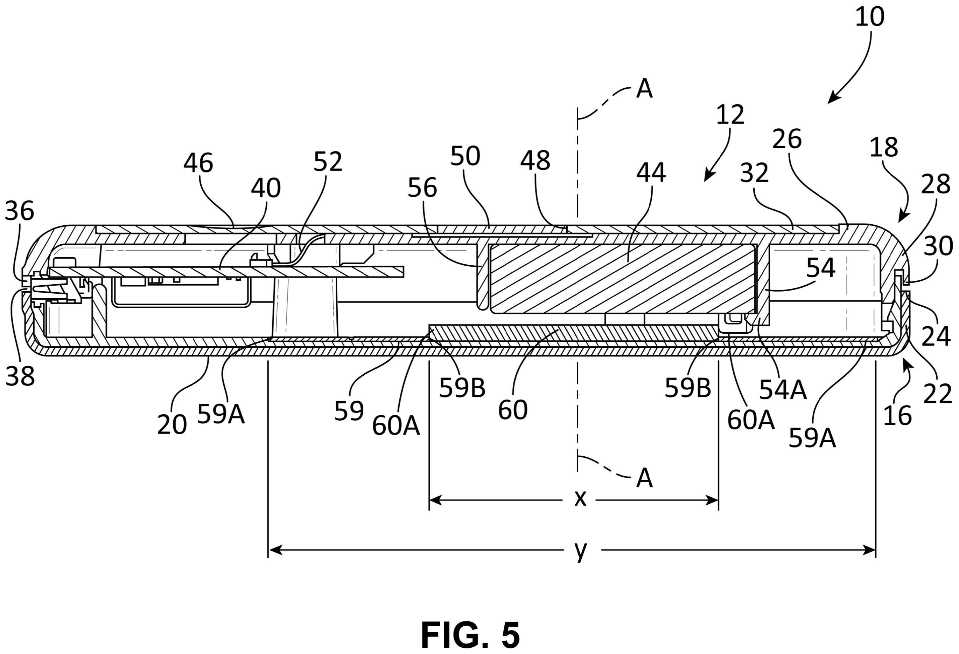

FIG. 5 illustrates an alternate embodiment of the present charger 10. In this embodiment, the charging coil 59 has a donut shape providing a central opening. The stack of ferrite sheets 60 is disposed in this opening. This means that the stack of ferrite sheets does not overlap the charging coil 59. However, as with the embodiment shown in FIG. 4, the charging coil 59 has a diameter of about 72 mm (radius = 36 mm) and the stack of ferrite sheets 60 has a diameter of about 37 mm. This means that the area of ferrite stack is about 1,075 mm² but the area of the charging coil 60 is about 2,997 mm² (4,072 mm² minus 1,075 mm²) giving a charging coil to ferrite stack area ratio of about 2.8. Again, an outer uncovered portion of the charging coil 59 extends inwardly from the outer coil edge 59A to the inner coil edge 59B with the inner coil edge being positioned immediately adjacent to and outwardly of the outer edge 60A of the ferrite sheet stack 60.

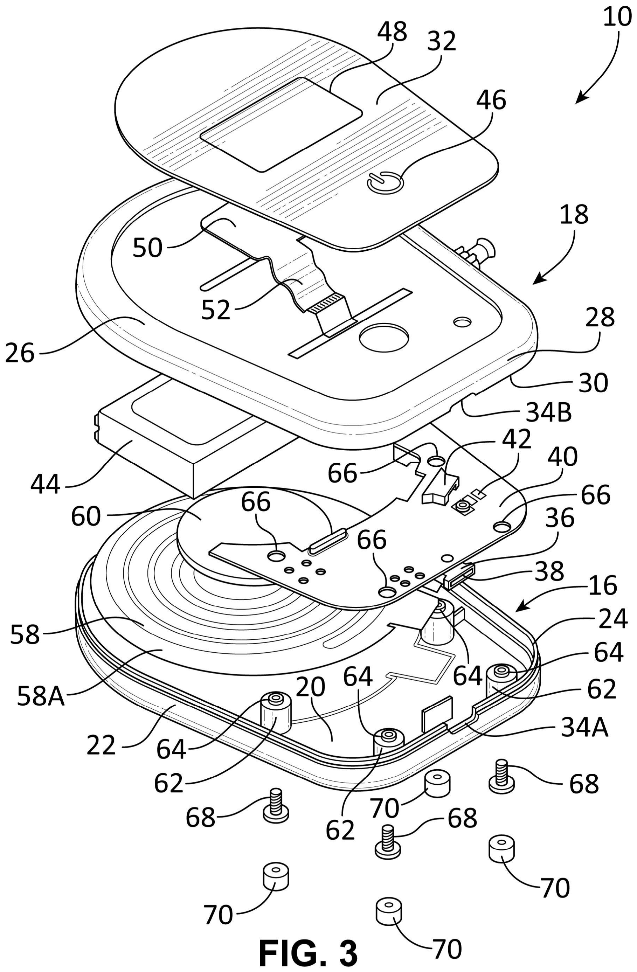

As shown in FIG. 3, the bottom clamshell 16 has a number of spaced-apart and upstanding posts 62 that are provided with raised detents 64. The raised detents 64 are sized to fit into similar sized openings 66 in the PCB assembly 40. With the PCB assembly 40 resting on the posts 62, the raised detents 64 extend into the PCB openings 66. While not shown in the drawings, the posts 62 have openings extending into the bottom wall 20 of the clamshell 16 that are sized to receive a threaded fastener 68 that is screwed through the openings 66 in the PCB assembly 40 and into treaded openings in the upper clamshell 18. This connects the bottom clamshell 16 to the upper clamshell 18 with the clamshell rims 24 and 30 aligned with each other and with the intermediate PCB assembly 40 secured in place, resting on the posts 62. Polymeric caps 70 are inserted into the fastener openings in the bottom clamshell 16 to cover the fasteners 68 and provide the wireless charger 10 of the present disclosure with an aesthetically pleasing appearance.

It is appreciated that various modifications to the inventive concepts described herein may be apparent to those skilled in the art without departing from the spirit and scope of the present invention as defined by the hereinafter appended claims.

## Claims

1. A wireless charger for an implantable medical device (IMD), the wireless charger comprising:
a) a housing forming an internal compartment;
b) a printed circuit board (PCB) assembly disposed in the internal compartment of the housing, wherein the PCB assembly comprises at least one electronic component that is configured to control the various functions of the wireless charger;
c) a battery disposed in the internal compartment of the housing, wherein the battery is connected to the PCB assembly to power the at least one electronic component;
d) a wound charging coil disposed in the internal compartment of the housing and being configured to output inductive power, wherein the charging coil is wound in a co-axial relationship around a longitudinal axis and extends to an outer coil edge providing the charging coil with a charging coil diameter; and
e) a stack comprising at least two ferrite sheets disposed in the internal compartment of the housing, wherein the stack of ferrite sheets is directly contacted to the charging coil.

2. The wireless charger of claim 1, wherein the stack of ferrite sheets has a stack outer edge providing a stack diameter, and wherein the stack diameter is less than the charging coil diameter so that a portion of the charging coil extending outwardly from the stack outer edge to the coil outer edge is not contacted by the stack of ferrite sheets.

3. The wireless charger of claim 1 or claim 2, wherein a charging coil to ferrite stack area ratio ranges from about 2 to 5.

4. The wireless charger of any of claims 1 to 3, wherein the stack of ferrite sheets is centered along the longitudinal axis.

5. The wireless charger of any of claims 1 to 4, wherein the charging coil has at least two turns of a copper trace formed in the PCB.

6. The wireless charger of any of claims 1 to 5, wherein the charging coil has a planar coil surface, and the stack of ferrite sheets is directly adhered to the planar coil surface.

7. The wireless charger of any of claims 1 to 6, wherein the at least two ferrite sheets comprising the stack of ferrite sheets are adhered together, or
wherein the stack of ferrite sheets has a stack thickness extending along the longitudinal axis that ranges from about 2.0 mm to about 3.0 mm, or
wherein the at least two ferrite sheets comprising the stack of ferrite sheets are comprised of porous NiCuZn ferrite, or
wherein each of the at least two ferrite sheets comprising the stack of ferrite sheets is about 0.3 mm thick.

8. The wireless charger of any of claims 1 to 7, wherein the battery is a rechargeable battery, and wherein the housing includes a charging port for selectively plugging in a charging cable to charge the rechargeable battery.

9. The wireless charger of any of claims 1 to 8, wherein the stack of ferrite sheets is aligned along the longitudinal axis between the battery and the charging coil.

10. A wireless charger for an implantable medical device (IMD), the wireless charger comprising:
a) a housing forming an internal compartment;
b) a printed circuit board (PCB) assembly disposed in the internal compartment of the housing, wherein the PCB assembly comprises at least one electronic component that is configured to control the various functions of the wireless charger;
c) a rechargeable battery disposed in the internal compartment of the housing, wherein the rechargeable battery is connected to the PCB assembly to power the at least one electronic component;
d) a wound charging coil disposed in the internal compartment of the housing and being configured to output inductive power, wherein the charging coil is wound in a co-axial relationship around a longitudinal axis and extends to an outer coil edge providing the charging coil with a charging coil diameter; and
e) a stack comprising at least two ferrite sheets disposed in the internal compartment of the housing, wherein the stack of ferrite sheets is directly adhered to the charging coil,
f) wherein the stack of ferrite sheets is centered along the longitudinal axis between the rechargeable battery and the charging coil.

11. The wireless charger of claim 10, wherein the stack of ferrite sheets has a stack outer edge providing a stack diameter, and wherein the stack diameter is less than the charging coil diameter so that a portion of the charging coil extending outwardly from the stack outer edge to the coil outer edge is not contacted by the stack of ferrite sheets.

12. The wireless charger of claim 10 or claim 11, wherein a charging coil to ferrite stack area ratio ranges from about 2 to 5.

13. The wireless charger of any of claims 10 to 12, wherein the charging coil has a planar coil surface, and the stack of ferrite sheets is directly adhered to the planar coil surface.

14. The wireless charger of any of claims 10 to 13, wherein the at least two ferrite sheets comprising the stack of ferrite sheets are adhered together, or
wherein the stack of ferrite sheets has a stack thickness extending along the longitudinal axis that ranges from about 2.0 mm to about 3.0 mm, or
wherein the at least two ferrite sheets comprising the stack of ferrite sheets are comprised of porous NiCuZn ferrite.

15. A wireless charger for an implantable medical device (IMD), the wireless charger comprising:
a) a housing forming an internal compartment;
b) a printed circuit board (PCB) assembly disposed in the internal compartment of the housing, wherein the PCB assembly comprises at least one electronic component that is configured to control the various functions of the wireless charger;
c) a battery disposed in the internal compartment of the housing, wherein the battery is connected to the PCB assembly to power the at least one electronic component;
d) a wound charging coil disposed in the internal compartment of the housing and being configured to output inductive power, wherein the charging coil has a donut-shape wound around a longitudinal axis and extends to an outer coil edge providing the charging coil with a charging coil diameter, wherein the donut-shaped charging coil has a central opening; and
e) a stack comprising at least two ferrite sheets disposed in the internal compartment of the housing, wherein the stack of ferrite sheets resides in the central opening of the donut-shaped charging coil so that the stack of ferrite sheets does not contact to the charging coil.
